# EUROPEAN PATENT SPECIFICATION

(11) **EP 3 681 428 B1**
(45) Date of publication and mention of the grant of the patent: **02.12.2026**
(21) Application number: 18793499.7
(22) Date of filing: 17.09.2018
(51) Int. Cl.: A61B 34/30, A61B 34/32, B25J 9/16

(54) **ROBOTIC CUTTING SYSTEMS FOR SURGICAL SAW BLADE CUTTING ON HARD TISSUE**
ROBOTISCHE SCHNEIDSYSTEME FÜR CHIRURGISCHES SÄGEBLATTSCHNEIDEN AUF HARTEM GEWEBE
SYSTÈMES DE COUPE ROBOTISÉS DE COUPE PAR LAME DE SCIE CHIRURGICALE SUR UN TISSU DUR

(30) Priority: 15.09.2017 US 201762559096 P
(43) Date of publication of application: 22.07.2020
(73) Proprietor: MAKO Surgical Corp., Weston, FL 33331 (US)
(72) Inventor: GILHOOLEY, Seamus, Athenry Galway (IE); BOWLING, David Gene, Albuquerque NM 87107 (US); SHIELS, Paul, Albuquerque NM 87110 (US)
(74) Representative: Röthinger, Rainer
(86) International application number: PCT/US2018/051313
(87) International publication number: WO 2019/055904

(56) References cited:
- EP-A1- 2 889 015
- EP-A2- 3 001 963
- WO-A1-2013/179053
- US-A1- 2009 287 222
- US-B2- 7 691 106

## Description

### Related Applications

This application claims the benefit of and priority to U.S. Provisional Patent Application No. 62/559,096, filed on September 15, 2017.

### Technical field

The embodiments and examples described herein generally relate to robotic cutting systems and methods of use thereof.

### Background

It is prevalent to use powered surgical saws during surgical procedures. Generally, these surgical saws may be operated by a user such as a surgeon or may be operated by a robotic cutting system. The surgical saws include a saw blade which is configured to cut hard tissue of a patient, such as bone. For example, saw blades are used in total knee arthroplasty, total hip arthroplasty, and similar types of procedures to create planar cuts on the bone.

In conventional surgical saws, undesirable skiving (e.g., deviation from an intended cut plane and/or deviation from an intended entry point) occurs during the cutting process, and specifically during the initial cut of the hard tissue with the saw blade. Skiving can be particularly difficult to control when making an initial cut on non-flat portions of hard tissue, such as at the ends of a femur (e.g., condyles, femur head). Skiving often includes undesirable flexing of the saw blade away from the desired location of the initial cut. One option to reduce skiving is to employ a cutting guide for the saw blade to hold the saw blade in place while making the necessary cuts. However, using cutting guides can increase the length of time it takes to make the necessary cuts because it requires that the cutting guide first be secured to the bone at the desired location. Additionally, the use of cutting guides often require the use of longer blades, which can still introduce skiving effects. In robotic surgery, one of the goals is to increase cutting accuracy and reduce cutting time, which can be difficult in cases where the saw blade is unable to initially cut at a desired location.

Document US 2009/287222 A1 discloses a method and a system for cutting the knee joint using a robot. A cantilevered cutter is introduced into the knee joint from inside and outside of upper and lower bones of the knee joint to realize a tunnel cutting technique in which cutting is conducted such that tunnels are defined in bone and remnant bone is cleared. The cutter includes a shaft in which a substantial axial portion is fitted into a sleeve and a remaining axial portion serves as a cantilever extending out of the sleeve, and a head, which is formed at a distal end of the shaft. A length of the cantilever and a diameter of the head are determined to have minimum sizes as long as the head can be introduced into bone in such a way as to define a tunnel and can be moved in the bone while cutting the bone.

Document EP 2 889 015 A1 discloses a handheld robot for orthopedic surgery and a control method thereof. The handheld robot includes a main body, a grip, a kinematic mechanism, a tool connector, a tool, a force sensor and a positioning unit. The handheld robot combines the position/orientation information of the tool acquired by the positioning unit with the force/torque information acquired by the force sensor, and utilizes the combined information to adjust the position of the tool so as to keep the tool within the range/path of a predetermined operation plan.

Document WO 2013/179053 A1 discloses a cranial drill system comprising one or more surgical drill elements for drilling into the brain and a cranial drilling system comprising a surgical drill element for drilling into the skull and an element for penetrating the dura.

Document US 7 691 106 B2 discloses a transverse acting saw blade for performing surgical cuts to bone tissue with minimal injury to surrounding tissue. The blade comprises an elongated member having a first portion and a second portion and a longitudinal and lateral axis. The first portion is configured to engage a drive source to produce longitudinal movement of the first portion that is atraumatic to surrounding tissue. The second portion includes a cutting surface. The longitudinal movement of the first portion is converted to a lateral movement of the second portion that is sufficient to cut engaged bone tissue with the cutting surface. The movement of the second portion is substantially transverse to the movement of the first portion.

A robotic cutting system for controlling a surgical saw in a manner that overcomes one or more of the aforementioned challenges is desired.

### Summary

According to aspects of the present invention, a robotic cutting system, another robotic cutting system and still another robotic cutting system are provided with the features of the independent claims. Preferred embodiments are mentioned in the dependent claims.

In one embodiment, a robotic cutting system is provided. The robotic cutting system comprises a robotic manipulator and a cutting tool to be coupled to the robotic manipulator. The cutting tool has an oscillating saw blade configured to cut a bone along a cutting plane. A controller is coupled to the robotic manipulator to control the robotic manipulator and the cutting tool to perform an initial stage of cutting along an initial plane prior to performing a final stage of cutting along the cutting plane. The initial plane is at a different orientation than the cutting plane. The controller is configured to control the robotic manipulator and the cutting tool to facilitate cutting along the initial plane with the saw blade to create a notch for the saw blade and to facilitate reorienting the saw blade from the initial plane to the cutting plane for performing the final stage of cutting along the cutting plane and wherein the notch is configured to capture the saw blade to limit skiving of the saw blade.

In an example useful for understanding the claimed invention, a robotic cutting system is provided. The robotic cutting system comprises a robotic manipulator and a cutting tool to be coupled to the robotic manipulator. The cutting tool has an oscillating saw blade configured to cut a bone along a cutting plane. A controller is coupled to the robotic manipulator to control the robotic manipulator to reciprocate the saw blade along the cutting plane to create a notch for the saw blade prior to resecting the bone along the cutting plane.

In an example useful for understanding the claimed invention, a robotic cutting system is provided. The robotic cutting system comprises a robotic manipulator and a cutting tool to be coupled to the robotic manipulator. The cutting tool has an oscillating saw blade configured to cut a bone along a cutting plane. The robotic cutting system also comprises a bur to be coupled to the robotic manipulator. A controller is coupled to the robotic manipulator to control the robotic manipulator to perform, with the bur, an initial stage of burring a notch into the bone for the blade prior to performing a final stage of cutting along the cutting plane with the saw blade.

In an example useful for understanding the claimed invention, a robotic cutting system is provided. The robotic cutting system comprises a robotic manipulator and a cutting tool to be coupled to the robotic manipulator. The cutting tool has a blade configured to cut a bone along a cutting plane. A controller is coupled to the robotic manipulator to control the robotic manipulator to perform cutting along the cutting plane. A blade guide is coupled to the robotic manipulator. The controller is configured to control the robotic manipulator and the cutting tool to facilitate cutting along the cutting plane while guiding the blade with the blade guide. The blade guide is capable of retracting as the blade penetrates into the bone along the cutting plane.

An example in form of a method of cutting bone using a robotic cutting system is also provided. The method, which is not claimed, comprises controlling a robotic manipulator and a cutting tool to facilitate cutting along an initial plane with a blade to create a notch for the blade and to facilitate reorienting the blade from the initial plane to a cutting plane, the initial plane being at a different orientation than the cutting plane.

Another example in form of a method of cutting bone using a robotic cutting system is provided. The method, which is not claimed, comprises controlling a robotic manipulator to reciprocate a blade along a cutting plane to create a notch for the blade prior to the bone being resected along the cutting plane.

Another example in form of a method of cutting bone using a robotic cutting system is provided. The method, which is not claimed, comprises controlling a robotic manipulator to burr a notch into the bone for receiving a blade prior to resecting the bone along a cutting plane with the blade.

Another example in form of a method of cutting bone using a robotic cutting system is provided. The method, which is not claimed, comprises controlling a robotic manipulator and a cutting tool to facilitate cutting along a cutting plane while guiding a blade with a retractable blade guide retracting the blade guard as the blade penetrates into the bone along the cutting plane.

### Brief Description of the Drawings

Advantages of the present disclosure will be readily appreciated as the same becomes better understood by reference to the following detailed description when considered in connection with the accompanying drawings wherein:

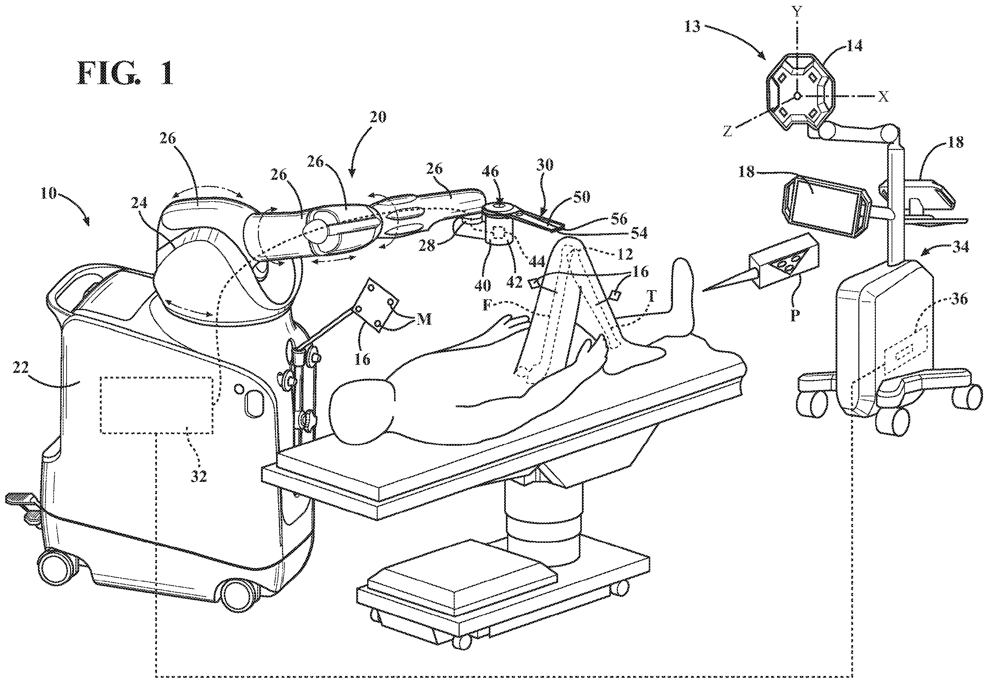

Figure 1 is a perspective view of a robotic cutting system in an operating room;
Figure 2 is a perspective view of the robotic cutting system;
Figure 3A is a side view of a cutting tool of the robotic cutting system according to one embodiment in a first orientation;
Figure 3B is a side view of the cutting tool of the robotic cutting system according to one embodiment in a second orientation;
Figure 4 is a side view of the cutting tool of the robotic cutting system according to another embodiment;
Figure 5A is a side view of the cutting tool of the robotic cutting system according to yet another embodiment;
Figure 5B is a side view of a blade of the robotic cutting system according to the embodiment illustrated in Figure 5A;
Figure 6A is a side view of the cutting tool of the robotic cutting system according to yet another embodiment in the first orientation;

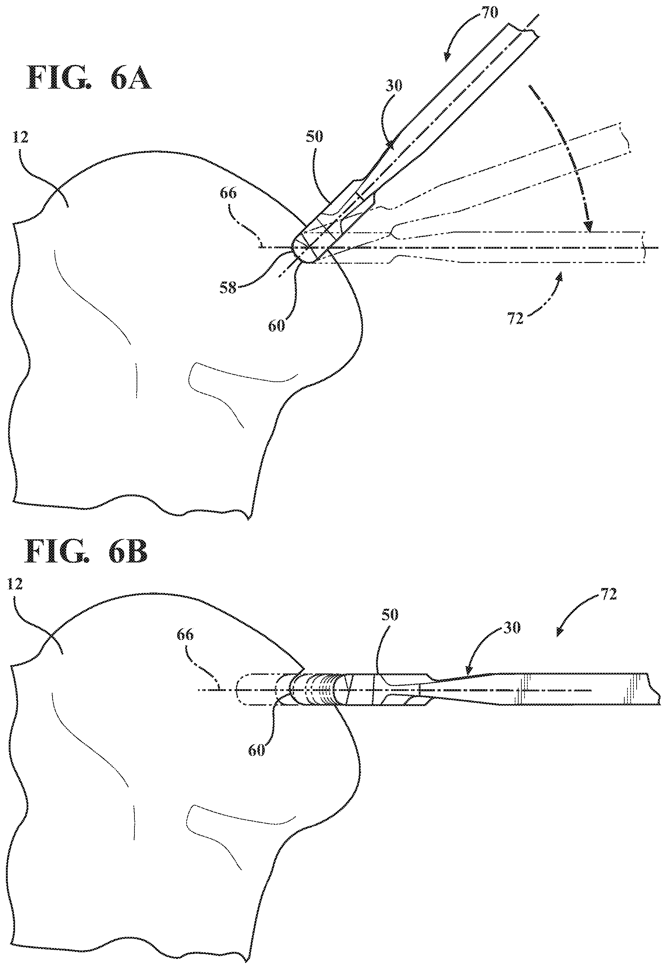

Figure 6A is a side view of the cutting tool of the robotic cutting system according to yet another embodiment in the first orientation;

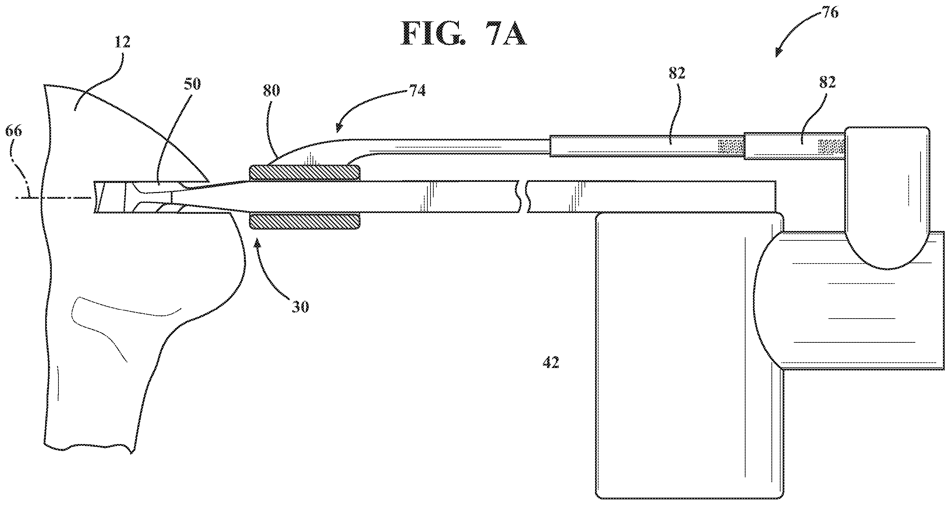

Figure 7A is a side view of the cutting tool of the robotic cutting system having a blade guide in an extended position; and

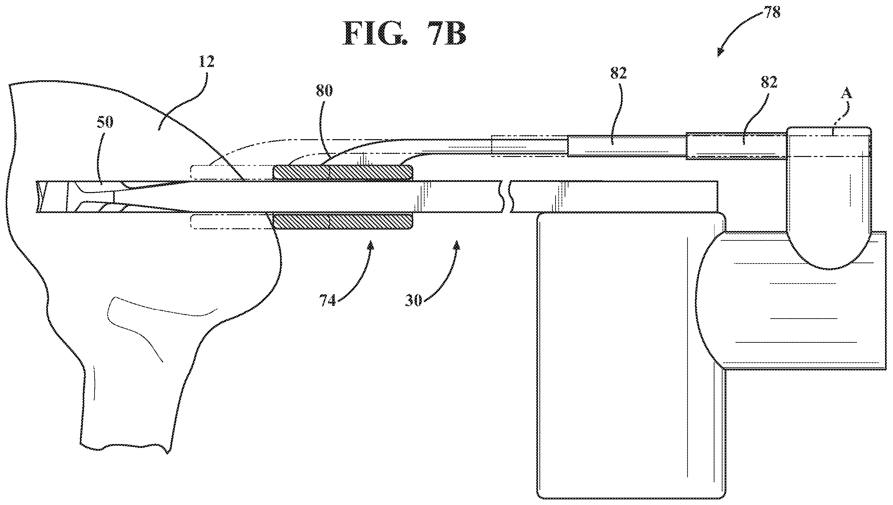

Figure 7B is a side view of the cutting tool of the robotic cutting system having the blade guide in a retracted position.

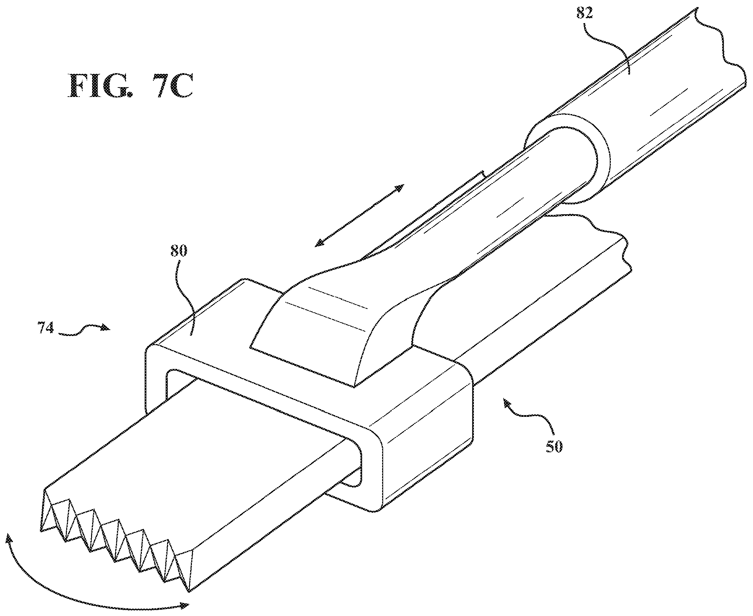

Figure 7C is a perspective view of the blade guide illustrating the blade guide defining a passage being wider than a saw blade to allow oscillations of the saw blade in the passage.

### Detailed Description

Referring to the Figures, a robotic cutting system 10 is shown for use during surgical procedures. The surgical procedures may be orthopedic surgeries, brain surgeries, or any other surgeries requiring the use of a cutting instrument. Typically, the surgical procedure will include the cutting of hard tissue 12, such as bone or the like. In some examples, the surgical procedure involves partial or total knee, hip, or shoulder replacement surgery, or may involve spine surgery.

The robotic cutting system 10 is designed to cut away material. In some cases, the material (e.g., bone) is to be replaced by surgical implants such as hip, knee, shoulder, and spine implants, including unicompartmental, bicompartmental, or total knee implants, acetabular cups, femur stems, humerus implants, and the like. Some of these types of implants are disclosed in U.S. Patent Application Publication No. 2012/0330429, entitled, "Prosthetic Implant and Method of Implantation,". It should be appreciated that the systems and methods disclosed herein may be used to perform other procedures, surgical or non-surgical, or may be used in industrial applications or other applications.

The robotic cutting system 10 comprises a navigation system 13 including a localizer 14 and tracking devices 16, one or more displays 18, and a robotic manipulator comprising a robotic arm 20 and a base 22. The robotic arm 20 includes a base link 24 rotatably coupled to the base 22 and a plurality of arm links 26 serially extending from the base link 24 to a distal end 28. The arm links 26 pivot/rotate about a plurality of joints in the robotic arm 20. A cutting tool 30 is connected to the distal end 28 of the robotic arm 20. The robotic arm 20 may be capable of moving the cutting tool 30 in multiple degrees of freedom, e.g., five or six degrees of freedom.

A robotic controller 32 is coupled to the robotic manipulator to provide control of the robotic arm 20 or guidance to the surgeon during manipulation of the cutting tool 30. In one embodiment, the robotic controller 32 is configured to control the robotic arm 20 (e.g., joint motors thereof) to provide haptic feedback to the user via the robotic arm 20. This haptic feedback helps to constrain or inhibit the surgeon from manually manipulating (e.g., moving) the cutting tool 30 beyond predefined virtual boundaries associated with the surgical procedure. Such a haptic feedback system and associated haptic objects that define the virtual boundaries are described, for example, in U.S. Patent No. 8,010,180. In one embodiment, the robotic cutting system 10 comprises the RIO^{™} Robotic Arm Interactive Orthopedic System manufactured by MAKO Surgical Corp. of Fort Lauderdale, FL, USA.

In some embodiments, the robotic arm 20 acts autonomously based on predefined tool paths and/or other predefined movements to perform the surgical procedure. Such movements may be defined during the surgical procedure and/or before the procedure. In further embodiments, a combination of manual and autonomous control is utilized. For example, a robotic system that employs both a manual mode in which a user manipulates the cutting tool 30 by applying force to the cutting tool 30 to cause movement of the robotic arm 20 and a semi-autonomous mode in which the user holds a pendant to control the robotic arm 20 to autonomously follow a tool path is described in U.S. Patent No. 9,566,122.

The navigation system 13 is set up to track movement of various objects in the operating room. Such objects include, for example, the cutting tool 30, the patient's anatomy of interest, e.g., the femur F and tibia T, and/or other objects. The navigation system 13 tracks these objects for purposes of displaying their relative positions and orientations to the surgeon and, in some cases, for purposes of controlling or constraining manual manipulation of the cutting tool 30 relative to virtual boundaries associated with the patient's anatomy.

The navigation system 13 includes a cart assembly 34 that houses a navigation controller 36. The navigation controller 36 and the robotic controller 32 collectively form a control system of the robotic cutting system 10. A navigation interface is in operative communication with the navigation controller 36. The navigation interface includes the displays 18 that are adjustably mounted to the cart assembly 34. Input devices such as a keyboard and mouse can be used to input information into the navigation controller 36 or otherwise select/control certain aspects of the navigation controller 36. Other input devices are contemplated including a touch screen (not shown) or voice-activation.

The localizer 14 communicates with the navigation controller 36. In the embodiment shown, the localizer 14 is an optical localizer and includes a camera unit (one example of a sensing device). The camera unit has an outer casing that houses one or more optical position sensors. In some embodiments at least two optical sensors are employed, sometimes three or more. The optical sensors may be separate charge-coupled devices (CCD). The camera unit is mounted on an adjustable arm to position the optical sensors with a field of view of the below discussed tracking devices 16 that, ideally, is free from obstructions. In some embodiments the camera unit is adjustable in at least one degree of freedom by rotating about a rotational joint. In other embodiments, the camera unit is adjustable about two or more degrees of freedom.

The localizer 14 includes a localizer controller (not shown) in communication with the optical sensors to receive signals from the optical sensors. The localizer controller communicates with the navigation controller 36 through either a wired or wireless connection (not shown). One such connection may be an IEEE 1394 interface, which is a serial bus interface standard for high-speed communications and isochronous real-time data transfer. The connection could also use a company specific protocol. In other embodiments, the optical sensors communicate directly with the navigation controller 36.

Position and orientation signals and/or data are transmitted to the navigation controller 36 for purposes of tracking the objects. The cart assembly 34, the displays 18, and the localizer 14 may be like those described in U.S. Pat. No. 7,725,162 to Malackowski, et al. issued on May 25, 2010, entitled "Surgery System,".

The navigation controller 36 can be a personal computer or laptop computer, or any other suitable form of controller. Navigation controller 36 has the displays 18, central processing unit (CPU) and/or other processors, memory (not shown), and storage (not shown).
may is distal end of an accessory of the cutting tool 30. Such accessories comprise an oscillating saw blade.

In the illustrated embodiment, the trackers 16 are passive trackers. In this embodiment, each tracker 16 has at least three passive tracking elements or markers M for reflecting light from the localizer 14 back to the optical sensors. In other embodiments, the trackers 16 are active trackers and may have light emitting diodes or LEDs transmitting light, such as infrared light to the optical sensors. Based on the received optical signals, navigation controller 36 generates data indicating the relative positions and orientations of the trackers 16 relative to the localizer 14. In some cases, more or fewer markers may be employed. For instance, in cases in which the object being tracked is rotatable about a line, two markers can be used to determine an orientation of the line by measuring positions of the markers at various locations about the line. It should be appreciated that the localizer 14 and trackers 16, although described above as utilizing optical tracking techniques, could alternatively, or additionally, utilize other tracking modalities to track the objects, such as electromagnetic tracking, radio frequency tracking, ultrasound tracking, inertial tracking, combinations thereof, and the like.

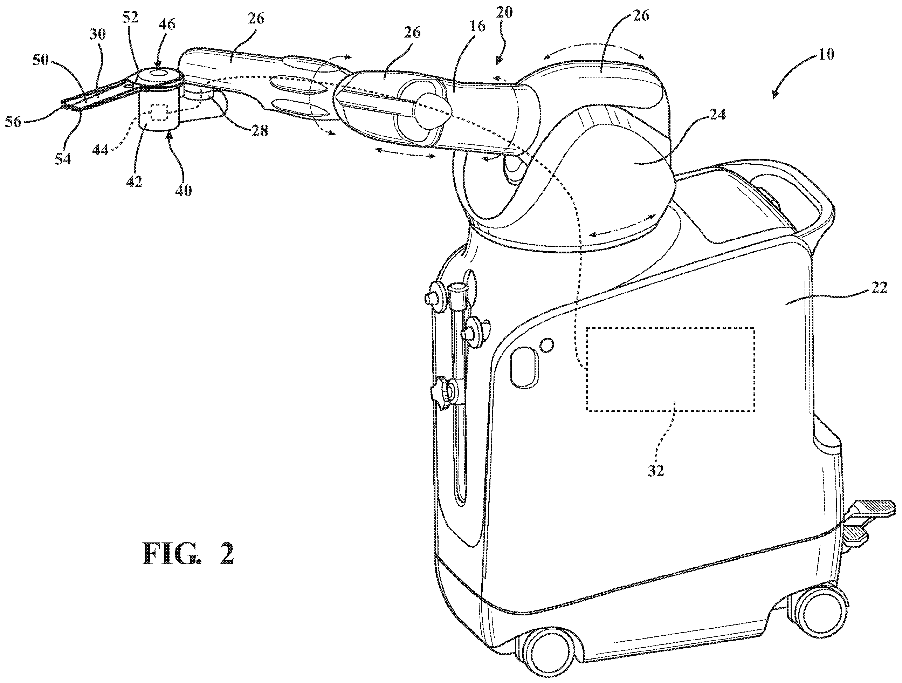

In some embodiments, such as the embodiment illustrated in Figure 2, the cutting tool 30 includes a coupler 40 having a housing 42 for attaching to the robotic manipulator. It is also contemplated that a separate motor 44 may be located in the housing 42 of the coupler 40. The motor 44 may be of any suitable type to operate the cutting tool 30, including but not limited to a pneumatic or electrical motor. The motor 44 is configured, for instance, to provide oscillating motion to the saw blade 50 of the cutting tool 30 during the surgical procedure. It is contemplated that the motor 44 may provide cyclical linear motion and/or cyclical angular motion, such as used for an oscillating sagittal saw. In some embodiments, the cutting tool 30 may include a drive hub 46 coupled to the motor 44.

The saw blade 50 may be of any size, shape, or type (i.e. straight blade, crescent blade, etc.). The saw blade 50 includes an attachment portion 52 configured to be removably coupled to the housing 42. Opposite the attachment portion 52, the saw blade 50 includes a cutting portion 54 which has a plurality of teeth 56. In some embodiments, the saw blade 50 is formed from a single piece of material, such as metal, by stamping and/or machining. The saw blade 50 may be configured to create a kerf with a generally flat face or may be configured to provide a kerf with a rounded profile. However, various configurations have been contemplated. The cutting tool 30 and associated saw blade 50 may be like that described in U.S. Patent Application Pub. No. 2017/0348007, filed on June 2, 2017, entitled "Surgical Saw and Saw Blade for use therewith,". The cutting tool 30 and associated saw blade 50 may also be like that described in U.S. Patent Application Pub. No. 2014/0180290, filed on December 21, 2012, entitled "Systems and Methods for Haptic Control of a Surgical Tool,".

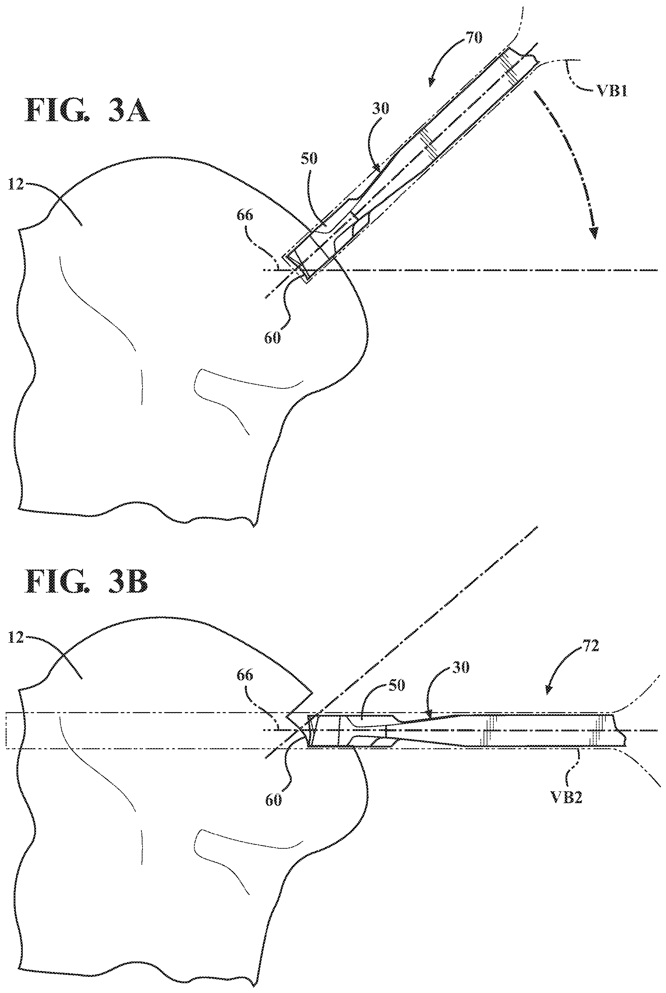

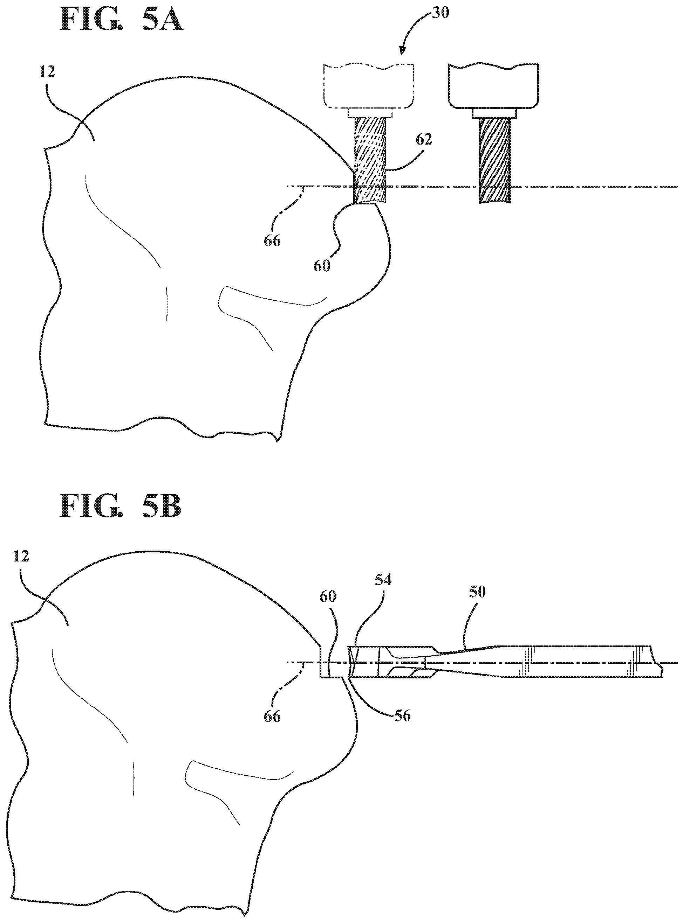

Referring now to Figures 3A through 7B, a desired cutting plane 66 is defined as the plane in which a planar cut is desired to be made with the saw blade 50. In the embodiments illustrated, the cutting plane 66 is disposed transverse to an outer surface of the hard tissue 12. More specifically, the outer surface of the hard tissue 12 (e.g., bone) is arcuate or curved such that the cutting plane 66 often extends non-perpendicularly from the outer surface. As a result, merely placing the saw blade 50 on the cutting plane 66, starting oscillation of the saw blade 50, and then making contact with the outer surface to initiate cutting, without more, will likely result in skiving of the saw blade 50 along the curved outer surface. The systems and methods described herein are intended to limit such skiving.

In these systems and methods, virtual objects (such as virtual boundaries), which may also be haptic objects (such as haptic boundaries), may be used to control (e.g., limit and/or constrain) movement of the saw blade 50 by operating the robotic arm in a desired manner to limit skiving. These virtual/haptic objects may be defined by points, lines, planes, volumes, or the like, and may be 1-D, 2-D, or 3-D. Such virtual/haptic objects may be defined as models and could be solid models (e.g., built with constructive solid geometry or the like), surface models (e.g., surface mesh, etc.), or any suitable form of 3-D model. These objects may be registered pre-operatively or intraoperatively to images/models (e.g., CT scans, X-ray images, MRI images, 3-D models, etc.) of the patient's anatomy that are mapped to the patient's actual anatomy using well-known registration techniques. Thus, in some embodiments, the locations of the virtual/haptic objects described herein are mapped to the patient's anatomy to control movement of the saw blade 50 in a manner that limits skiving of the saw blade 50. For example, the robotic cutting system 10 may be controlled based on a haptic boundary that defines a desired plane in which the saw blade 50 should be constrained. In this case, the robotic controller 32 operates the robotic arm 20 so that the saw blade is confined by the haptic boundary to stay on the desired plane. The manner of controlling the robotic cutting system, e.g., the robotic arm 20, based on such virtual/haptic objects is described, for example, in U.S. Patent No. 8,010,180, U.S. Patent No. 9,119,655, or U.S. Patent Application Pub. No. 2014/0180290.

In some procedures, such as during a total knee procedure, several planar cuts are made to the hard tissue 12, and any of these planar cuts may employ the methods described herein. In some examples, cutting may be completely through the hard tissue 12 or only partially through the hard tissue 12 such that the cut is finished when a pre-determined final depth is reached. The cutting plane 66 may be defined pre-operatively by the surgeon, such as by defining desired planar cuts on a virtual 3-D model of the hard tissue 12 created using pre-operative images taken of the hard tissue. The desired planar cuts may also be defined by the shape of the implant and a 3-D model of the implant. The cutting plane 66 may be defined intraoperatively by the surgeon, or automatically by the control system. A position and orientation of the cutting plane 66 may be tracked by the navigation system 13 as the hard tissue 12 moves during the surgical procedure by virtue of the tracker 16 attached to the hard tissue 12 and registration of the tracker 16 to the hard tissue 12. The location of the cutting plane 66 may be tracked by virtue of being mapped to the 3-D model that includes the cutting plane 66. The robotic manipulator can accommodate movement of the cutting plane 66 and autonomously adjust its own positioning as needed to maintain any desired relationship to the hard tissue 12 required in the methods described herein, such as staying on a desired plane with respect to the hard tissue 12 when necessary. Such control may be accomplished using the robotic controls described, for example, in U.S. Patent No. 8,010,180, U.S. Patent No. 9,119,655, or U.S. Patent Application Pub. No. 2014/0180290.

Referring to Figures 3A and 3B, one system and method for limiting skiving of the saw blade 50 is illustrated. In this example, the robotic cutting system 10 begins the cut with the saw blade 50 in a first orientation 70 which is normal to the outer surface of the hard tissue 12 in the vicinity of the cutting plane 66. The saw blade 50 remains in the first orientation 70 until the cut reaches a predetermined depth. It is also contemplated that the first orientation 70 may be any position not in-line with the cutting plane 66 that provides an advantage in reducing skiving of the saw blade 50. The predetermined depth may be any depth, but is typically enough depth to create an initial notch 60 (also referred to as a groove) in the hard tissue 12. Once the notch 60 is created, the saw blade 50 is reoriented (e.g., articulated) to a second orientation 72 (see Figure 3B), in line with the cutting plane 66, and the cut is finished. The notch 60 is used to create a fulcrum in the hard tissue 12 about which the saw blade 50 is able to pivot from the first orientation 70 to the second orientation 72.

In operation, the cutting tool 30 is first coupled to the robotic manipulator. The control system is configured to control movement of the cutting tool 30 via the robotic manipulator. The control system may comprise a tool controller for operating the motor 44 of the cutting tool 30 to facilitate cutting. The tool controller may comprise part of the robotic controller 32, or be separate from the robotic controller 32. When the cutting tool 30 is coupled to the robotic manipulator, and the user is ready to begin cutting along the cutting plane 66, the control system will send a command to the robotic arm 20 (e.g., to control the joint motors thereof) to move the cutting tool 30 so that the saw blade 50 is first located on (e.g., aligned with) an initial plane associated with the first orientation 70 (Figure 3A). The saw blade 50 is aligned with the initial plane by being in the same general orientation as the initial plane with the initial plane passing through or being adjacent to the saw blade 50. In other embodiments, the user may position the cutting tool 30 at the first orientation 70 by virtue of haptic guidance as described herein. The user may be able to operate the robotic cutting system 10 to move the cutting tool 30 so that the saw blade 50 is automatically aligned with the initial plane in the first orientation in the manner described in U.S. Patent Application Pub. No. 2014/0180290. The robotic cutting system 10, by virtue of the navigation system 13 and associated trackers 16, and/or by virtue of the encoders in the joints of the robotic arm 20, is able to determine the position and orientation of the saw blade 50 with respect to the initial plane to locate the saw manipulator in the same manner described in U.S. Patent No. 8,010,180, .

One example of a virtual boundary VB1 to align the saw blade 50 with the initial plane is shown in Figure 3A in the form of a bounded volume that defines the initial plane. The virtual boundary VB1 has a thickness about the thickness of the saw blade 50 to constrain the saw blade 50 from moving away from the first orientation 70, a width (not shown) which is about the same as the width of the saw blade 50 (when oscillating) to avoid any unnecessary lateral movement, an open starting end (so that the saw blade 50 can enter the virtual boundary VB1), and a closed target end at the desired depth of the notch 60. The virtual boundary VB1 can be mapped with respect to the hard tissue 12 pre-operatively or intra-operatively using registration techniques for mapping the model of the virtual boundary VB1 to the 3-D model of the hard tissue 12. Alternatively, the user could locate the virtual boundary VB1 manually on a user interface connected to the navigation controller 36. Such virtual boundaries may also be haptic boundaries that operate to limit or constrain movement of the saw blade 50 and provide haptic feedback to the user in the event the saw blade 50 is being moved in a manner that violates, or will violate, the boundary.

Once the predetermined depth is reached and the initial notch 60 is formed, the saw blade 50 is autonomously reoriented by the robotic manipulator (or manually by the user) to the second orientation 72 in line with the cutting plane 66, as illustrated in Figure 3B. Thus, the virtual boundary VB1 may be deactivated and a separate virtual boundary associated with the second orientation 72 may be activated to keep the saw blade 50 on the cutting plane 66 during manual cutting. The saw blade 50 then finishes the cut through the hard tissue 12 along the cutting plane 66.

It is contemplated that the saw blade 50 may continue cutting during reorientation from the first orientation 70 to the second orientation 72 such that the notch 60 has an arcuate shape. In this case, the teeth of the saw blade 50 may be arranged to cut the hard tissue 12 in multiple directions, including in the direction of reorientation to form the notch 60. However, it is also contemplated that the blade 50 may be removed from the hard tissue 12 and then reoriented to the second orientation 72 before finishing the cut along the cutting plane 66 - in this case a small ridge of bone left from cutting along the initial plane may first be encountered by the saw blade 50 before it reaches the notch 60, which may cause a slight deflection of the saw blade 50, but then the notch 60 acts to capture the saw blade 50 and keep it on the cutting plane 66.

To facilitate movement from the first orientation 70 and the initial plane to the second orientation 72 and the cutting plane 66, an intermediate virtual boundary may be provided that extends from the initial plane to the cutting plane so that the user in unable to move the saw blade 50 beyond either plane, but is allowed to freely reorient the saw blade 50 manually. In one version, the distal end of the saw blade 50 pivots in the notch 60 from the first orientation and the initial plane to the second orientation and the cutting plane. The robotic cutting system 10 may also operate to automatically move the saw blade 50 from the initial plane to the cutting plane 66 in the same manner that the control system in U.S. Patent Application Pub. No. 2014/0180290 automatically aligns the saw blade. Thus, such motion may be manual or autonomous. Another virtual boundary may be activated once the saw blade 50 is aligned with the cutting plane 66 to restrict movement of the saw blade 50 to along the cutting plane 66. This virtual boundary may also be a haptic boundary that limits or constrains movement of the saw blade 50 to the cutting plane 66. An example of this virtual boundary is illustrated as virtual boundary VB2 in Figure 3B, which is shown as a volume, similar to the virtual boundary VB1.

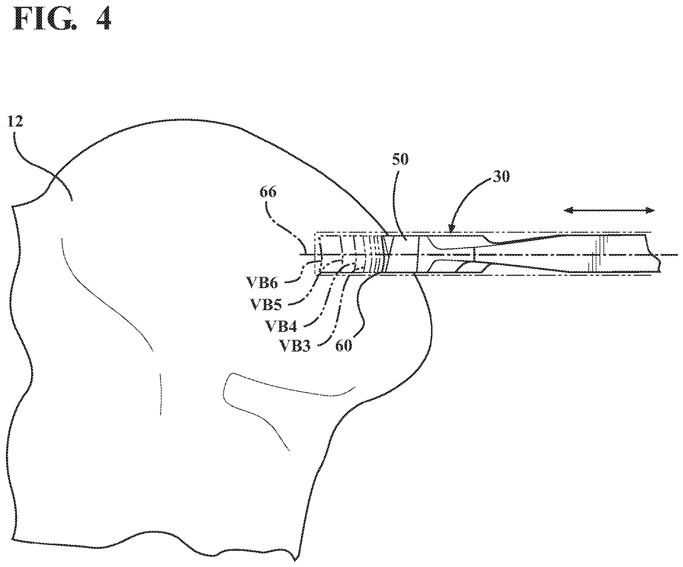

In another embodiment, illustrated in Figure 4, the cutting tool 30 is initially placed in line with the cutting plane 66 (either autonomously or manually with virtual objects). For instance, the user may be able to operate the robotic cutting system 10 to move the cutting tool 30 so that the saw blade 50 is automatically aligned with the cutting plane 66 in the manner described in U.S. Patent Application Pub. No. 2014/0180290. Once on the cutting plane 66, the cutting tool 30 is reciprocated to form the initial notch 60 in the hard tissue 12. This reciprocating motion (also referred to as pecking) may be carried out autonomously or manually as well. This reciprocating motion may be defined as motion axially along a longitudinal axis of the blade, e.g., reciprocating lengthwise. If carried out autonomously, the robotic manipulator is configured to move the cutting tool 30 and its saw blade 50 along the cutting plane 66 at incrementally increasing depth, in combination with slight retractions, to effectively chip away at the outer surface of the hard tissue 12 to limit skiving that could otherwise occur if attempting to start cutting at a non-perpendicular orientation to the curved surface. In this case, the autonomous movement creates the reciprocating motion needed to form the initial notch 60 in the hard tissue 12 such that the saw blade 50 is capable of being captured in the notch to limit skiving. The saw blade 50 may be constrained to stay on the cutting plane 66 via one or more virtual boundaries associated with each of the reciprocations (e.g., the virtual boundary dynamically changes to increase the depth as hard tissue is removed, or new virtual boundaries are created after each contact made with the hard tissue 12). An example of an initial virtual boundary VB3 for the first peck at the hard tissue 12 and sequentially created virtual boundaries VB4, VB5, VB6 for subsequent pecks at the hard tissue 12 are shown in Figure 4.

## Claims

1. A robotic cutting system (10) comprising:
a robotic manipulator (20);
a cutting tool (30) to be coupled to the robotic manipulator (20), the cutting tool (30) having an oscillating saw blade (50) configured to cut a bone along a cutting plane (66); and
a controller (32, 36) coupled to the robotic manipulator (20) to control the robotic manipulator (20) and the cutting tool (30) to:
perform an initial stage of cutting along an initial plane prior to performing a final stage of cutting along the cutting plane (66), the initial plane being at a different orientation than the cutting plane (66), wherein the controller (32, 36) is configured to control the robotic manipulator and the cutting tool to facilitate cutting along the initial plane with the saw blade (50) to create a notch (60) for the saw blade (50); and
facilitate reorienting the saw blade (50) from the initial plane to the cutting plane (66) for performing the final stage of cutting along the cutting plane (66) and wherein the notch (60) is configured to capture the saw blade (50) to limit skiving of the saw blade (50).

2. The robotic cutting system (10) of claim 1, wherein the controller (32, 36) is configured to control the robotic manipulator (20) and the cutting tool (30) to facilitate reorienting the saw blade (50) while the saw blade (50) is located in the notch (60).

3. The robotic cutting system (10) of claim 2, wherein the controller (32, 36) is configured to control the robotic manipulator (20) and the cutting tool (30) to facilitate reorienting the saw blade (50) while the saw blade (50) is oscillating.

4. The robotic cutting system (10) of any preceding claim, wherein the initial plane is defined by a first virtual boundary (VB) and the controller (32, 36) is configured to control the robotic manipulator (20) to perform the initial stage of cutting along the initial plane by constraining movement of the saw blade (50) using the first virtual boundary (VB).

5. The robotic cutting system (10) of claim 4, wherein the cutting plane (66) is defined by a second virtual boundary (VB), different than the first virtual boundary (VB), and the controller (32, 36) is configured to deactivate the first virtual boundary (VB) after forming the notch (60).

6. The robotic cutting system (10) of any preceding claim, wherein the controller (32, 36) is configured to control the robotic manipulator (20) and the cutting tool (30) to facilitate cutting along the initial plane by at least one of: autonomously moving the cutting tool (30); and constraining movement of the cutting tool (30).

7. The robotic cutting system (10) of any preceding claim, wherein the controller (32, 36) is configured to control the robotic manipulator (20) and the cutting tool (30) to reorient the saw blade (50) from the initial plane to the cutting plane (66) by at least one of: autonomously moving the cutting tool (30); and constraining movement of the cutting tool (30).

8. The robotic cutting system (10) of any preceding claim, wherein the controller (32, 36) is configured to autonomously align the saw blade (50) with the initial plane.

9. The robotic cutting system (10) of any preceding claim, wherein the initial plane is defined as being normal to an outer surface of the bone.

10. The robotic cutting system (10) of any preceding claim, wherein the saw blade (50) has a rounded cutting end.

## Patentansprüche

1. Roboterschneidsystem (10), umfassend:
einen Robotermanipulator (20);
ein Schneidwerkzeug (30), das mit dem Robotermanipulator (20) koppelbar ist, wobei das Schneidwerkzeug (30) ein oszillierendes Sägeblatt (50) aufweist, das ausgebildet ist, um einen Knochen entlang einer Schnittebene (66) zu schneiden; und
eine Steuerung (32, 36), die mit dem Robotermanipulator (20) gekoppelt ist, um den Robotermanipulator (20) und das Schneidwerkzeug (30) derart zu steuern, dass:
vor der Durchführung einer abschließenden Schnittphase entlang der Schnittebene (66) eine anfängliche Schnittphase entlang einer Anfangsebene durchgeführt wird, wobei die Anfangsebene in einer anderen Ausrichtung als die Schnittebene (66) liegt, wobei die Steuerung (32, 36) ausgebildet ist, um den Robotermanipulator und das Schneidwerkzeug derart zu steuern, dass das Schneiden entlang der Anfangsebene mit dem Sägeblatt (50) ermöglicht wird, um eine Kerbe (60) für das Sägeblatt (50) zu erzeugen; und
eine Neuausrichtung des Sägeblatts (50) von der Anfangsebene auf die Schnittebene (66) zu ermöglichen, um die abschließende Schnittphase entlang der Schnittebene (66) durchzuführen, und wobei die Kerbe (60) ausgebildet ist, um das Sägeblatt (50) aufzunehmen und so ein Abwandern des Sägeblatts (50) zu begrenzen.

2. Roboterschneidsystem (10) nach Anspruch 1, wobei die Steuerung (32, 36) ausgebildet ist, um den Robotermanipulator (20) und das Schneidwerkzeug (30) zu steuern, um die Neuausrichtung des Sägeblatts (50) zu ermöglichen, während sich das Sägeblatt (50) in der Kerbe (60) befindet.

3. Roboterschneidsystem (10) nach Anspruch 2, wobei die Steuerung (32, 36) ausgebildet ist, um den Robotermanipulator (20) und das Schneidwerkzeug (30) zu steuern, um die Neuausrichtung des Sägeblatts (50) zu ermöglichen, während das Sägeblatt (50) oszilliert.

4. Roboterschneidsystem (10) nach einem der vorhergehenden Ansprüche, wobei die Anfangsebene durch eine erste virtuelle Begrenzung (VB) definiert ist und die Steuerung (32, 36) ausgebildet ist, um den Robotermanipulator (20) zu steuern, um die Anfangsphase des Schneidvorgangs entlang der Anfangsebene durchzuführen, indem sie die Bewegung des Sägeblatts (50) unter Verwendung der ersten virtuellen Begrenzung (VB) einschränkt.

5. Roboterschneidsystem (10) nach Anspruch 4, wobei die Schnittebene (66) durch eine zweite virtuelle Begrenzung (VB) definiert ist, die sich von der ersten virtuellen Begrenzung (VB) unterscheidet, und die Steuerung (32, 36) ausgebildet ist, um die erste virtuelle Begrenzung (VB) nach dem Bilden der Kerbe (60) zu deaktivieren.

6. Roboterschneidsystem (10) nach einem der vorhergehenden Ansprüche, wobei die Steuerung (32, 36) ausgebildet ist, um den Robotermanipulator (20) und das Schneidwerkzeug (30) zu steuern, um das Schneiden entlang der Anfangsebene durch ein autonomes Bewegen des Schneidwerkzeugs (30) und/oder ein Einschränken der Bewegung des Schneidwerkzeugs (30) zu ermöglichen.

7. Roboterschneidsystem (10) nach einem der vorhergehenden Ansprüche, wobei die Steuerung (32, 36) ausgebildet ist, um den Robotermanipulator (20) und das Schneidwerkzeug (30) zu steuern, um das Sägeblatt (50) von der Anfangsebene zur Schnittebene (66) neu auszurichten, durch: ein autonomes Bewegen des Schneidwerkzeugs (30) und/oder ein Einschränken einer Bewegung des Schneidwerkzeugs (30).

8. Roboterschneidsystem (10) nach einem der vorhergehenden Ansprüche, wobei die Steuerung (32, 36) ausgebildet ist, um das Sägeblatt (50) autonom auf die Anfangsebene auszurichten.

9. Roboterschneidsystem (10) nach einem der vorhergehenden Ansprüche, wobei die Anfangsebene als senkrecht zu einer Außenfläche des Knochens liegend definiert ist.

10. Roboterschneidsystem (10) nach einem der vorhergehenden Ansprüche, wobei das Sägeblatt (50) ein abgerundetes Schneidende aufweist.

## Revendications

1. Système de coupe robotisé (10) comprenant :
un manipulateur robotique (20);
un outil de coupe (30) destiné à être couplé au manipulateur robotisé (20), l'outil de coupe (30) comportant une lame de scie oscillante (50) conçue pour couper un os le long d'un plan de coupe (66); et
un organe de commande (32, 36) couplé au manipulateur robotisé (20) pour commander le manipulateur robotisé (20) et l'outil de coupe (30) pour:
effectuer une étape initiale de coupe le long d'un plan initial avant d'effectuer une étape finale de coupe le long du plan de coupe (66), le plan initial ayant une orientation différente de celle du plan de coupe (66), dans lequel l'organe de commande (32, 36) est configuré pour amener le manipulateur robotisé et l'outil de coupe à faciliter la coupe le long du plan initial à l'aide de la lame de scie (50) pour pratiquer une encoche (60) destinée à la lame de scie (50); et
faciliter la réorientation de la lame de scie (50) à partir du plan initial vers le plan de coupe (66) afin d'effectuer la phase finale de la coupe le long du plan de coupe (66), et dans lequel l'encoche (60) est conçue pour retenir la lame de scie (50) afin de limiter la dérivation de la lame de scie (50).

2. Système de coupe robotisé (10) selon la revendication 1, dans lequel l'organe de commande (32, 36) est configuré pour amener le manipulateur robotisé (20) et l'outil de coupe (30) à faciliter la réorientation de la lame de scie (50) lorsque la lame de scie (50) se trouve dans l'encoche (60).

3. Système de coupe robotisé (10) selon la revendication 2, dans lequel l'organe de commande (32, 36) est configuré pour amener le manipulateur robotisé (20) et l'outil de coupe (30) à faciliter la réorientation de la lame de scie (50) pendant que la lame de scie oscille.

4. Système de coupe robotisé (10) selon l'une quelconque revendication précédente, dans lequel le plan initial est défini par une première limite virtuelle (VB) et l'organe de commande (32, 36) est configuré pour amener le manipulateur robotisé (20) à effectuer l'étape initiale de coupe le long du plan initial en limitant le mouvement de la lame de scie (50) à l'aide de la première limite virtuelle (VB).

5. Système de coupe robotisé (10) selon la revendication 4, dans lequel le plan de coupe (66) est défini par une deuxième limite virtuelle (VB), différente de la première limite virtuelle (VB), et l'organe de commande (32, 36) est configuré pour désactiver la première limite virtuelle (VB) après la formation de l'encoche (60).

6. Système de coupe robotisé (10) selon l'une quelconque revendication précédente, dans lequel l'organe de commande (32, 36) est configuré pour amener le manipulateur robotisé (20) et l'outil de coupe (30) à faciliter la coupe le long du plan initial par au moins l'une opération parmi le déplacement autonome de l'outil de coupe (30) ; et la limitation du mouvement de l'outil de coupe (30).

7. Système de coupe robotisé (10) selon l'une quelconque revendication précédente, dans lequel l'organe de commande (32, 36) est configuré pour amener le manipulateur robotisé (20) et l'outil de coupe (30) à réorienter la lame de scie (50) du plan initial vers le plan de coupe (66) par au moins une opération parmi le déplacement autonome de l'outil de coupe (30) ; et la limitation du mouvement de l'outil de coupe (30).

8. Système de coupe robotisé (10) selon l'une quelconque revendication précédente, dans lequel l'organe de commande (32, 36) est configuré pour aligner de manière autonome la lame de scie (50) sur le plan initial.

9. Système de coupe robotisé (10) selon l'une quelconque revendication précédente, dans lequel le plan initial est défini comme étant perpendiculaire à une surface externe de l'os.

10. Système de coupe robotisé (10) selon l'une quelconque revendication précédente, dans lequel la lame de scie (50) présente une extrémité de coupe arrondie.
